**Europäisches Patentamt**

**European Patent Office**

**Office européen des brevets**

(11) Veröffentlichungsnummer: **0 147 755**
**B1**

# EUROPÄISCHE PATENTSCHRIFT

(45) Veröffentlichungstag der Patentschrift:
05.07.89

(51) Int. Cl.⁴: **A 61 M 15/00**

(21) Anmeldenummer: **84115536.9**

(22) Anmeldetag: **15.12.84**

(54) Inhalator.

(30) Priorität: **17.12.83 DE 3345722**

(43) Veröffentlichungstag der Anmeldung:
**10.07.85 Patentblatt 85/28**

(45) Bekanntmachung des Hinweises auf die Patenterteilung:
**05.07.89 Patentblatt 89/27**

(84) Benannte Vertragsstaaten:
**AT BE CH DE FR IT LI LU NL SE**

(56) Entgegenhaltungen:
**US-A- 2 569 720**
**US-A- 4 069 819**

(73) Patentinhaber: **BOEHRINGER INGELHEIM KG,**
**Postfach 200, D-6507 Ingelheim am Rhein (DE)**

(72) Erfinder: **Kladders, Heinrich, Ulmenstrasse 3,**
**D-6507 Ingelheim am Rhein (DE)**

## Beschreibung

Die Erfindung betrifft einen Inhalator für die Inhalation pulverförmiger, insbesondere mikronisierter Arzneimittel aus üblichen Kapseln, bei dem die geöffneten Kapseln durch einen Luftstrom während des Inhalationsvorgangs in Vibration versetzt werden. Die Kapseln bestehen üblicherweise aus Hartgelatine. Das Verhältnis zwischen ihrer Länge und ihrem Durchmesser beträgt im allgemeinen 2 : 1 bis 3 : 1, kann jedoch zwischen etwa 1,5 : 1 und 4 : 1 liegen.

Der erfindungsgemäße Inhalator besitzt eine längliche Kapselkammer, deren lichte Weite 1,1- bis 2,5mal so groß ist wie der Kapseldurchmesser und deren Länge 1,02- bis 2mal so groß ist wie die Länge der Kapsel, und weist einen Lufteinlaß im Boden der Kapselkammer und einen Luftauslaß in der Decke der Kapselkammer auf, so daß der Luftstrom axial geführt wird; der neue Inhalator ist dadurch gekennzeichnet, daß die Kapselkammer von einem Unterteil, das mit einer Schneidvorrichtung mit zwei Spitzen bzw. Schneiden versehen ist, und einem Mundstück begrenzt wird, wobei Unterteil und Mundstück so ausgebildet sind, daß sie aus der Normalposition axial gegeneinander in die Lochungsposition verschoben werden können, in der die Kapsel fixiert ist und durch Betätigung der Schneidvorrichtung seitlich nahe den beiden Kapselenden gelocht bzw. aufgeschnitten wird.

Es sind bereits Inhalatoren für die Inhalation in Kapseln abgefüllter Arzneimittel beschrieben worden, bei denen die Kapseln während der Inhalation bewegt werden. Ein Inhalator dieser Art ist aus der DE-A-1 566 604 bekannt. Der dort beschriebene Inhalator verfügt über eine koaxial mit der Längsachse des Geräts angeordnete, mit propellerartigen Flügeln versehene Einrichtung zur Aufnahme der Kapsel. Beim Inhalieren durch das Gerät wird die Einrichtung mit der Kapsel in Rotation versetzt.

Während bei dem vorstehend beschriebenen Inhalator die Arzneistoffkapsel in einem beweglichen Teil des Geräts fixiert ist und mit diesem bewegt wird, beschreibt die FR-A-2 146 202 ein Inhalationsgerät mit einer flachen zylindrischen Kammer, in der nur die Kapsel selbst sich bewegt. Die an den Enden geöffnete Kapsel rotiert beim Inhalationsvorgang, durch tangential einströmende Luft angetrieben, um ihre Querachse.

Beide bekannten Inhalatoren zeigen vor allem den Nachteil, daß die Ausbringung des Arzneimittels ungleichmäßig ist. Manche Kapseln werden praktisch vollständig geleert, andere enthalten nach dem Inhalieren noch erhebliche Anteile des Arzneimittels. Dies ist besonders bei sehr feinteiligen (mikronisierten) Arzneimitteln der Fall.

Aus der US-A-2 569 720 ist ein Inhalator bekannt, der im Boden der Kapselkammer eine zentrale Lufteinlaßöffnung aufweist und bei dem die Decke der Kapselkammer als siebförmiger Luftauslaß ausgeführt ist. Der Inhalator wird für die Inhalation von Pulvern aus Kapselunterteilen vorgeschlagen, d.h. die mit dem Pulver gefüllten (Steck-) Kapseln müssen außerhalb des Inhalators auseinandergezogen und dann in die Kapselkammer eingelegt werden. Dort soll das Kapselunterteil durch den Luftstrom in starke Bewegung versetzt werden. Insbesondere wird ein überschlagen des Kapselunterteils als vorteilhaft angesehen. Für Asthmapatienten, für die Inhalatoren der beschriebenen Art vor allem bestimmt sind, ist es – besonders unter einem Asthmaanfall – unzumutbar, in der angegebenen Weise zu verfahren, da es leicht zum Verschütten von Arzneipulver und damit zu unzureichender Dosierung kommen kann.

In der US-A-4 069 819 ist ein Inhalator beschrieben, bei dem die Kapsel durch den Boden der Kapselkammer aufgestochen und während der Inhalation durch die in der Nähe des Bodens tangential eintretende Luft in Bewegung versetzt wird. Nachbau und Prüfung des beschriebenen Inhalators zeigten, daß die Wirkstoffausbringung ungleichmäßig und sehr unvollständig (unter 40%) ist, während mit dem erfindungsgemäßen Inhalator bei Verwendung der gleichen Kapseln und Pulver regelmäßig eine Entleerung von über 90% erreicht wurde.

Der erfindungsgemäße Inhalator ermöglicht nicht nur eine verläßlichere Ausbringung des Arzneimittels mit geringer Standardabweichung, er hat auch den zusätzlichen Vorteil der besseren Desagglomeration. Die mikronisierten Arzneimittel in den Kapseln neigen nämlich zur Bildung von Agglomeraten. Diese Agglomerate sind therapeutisch unerwünscht, denn es wird eine möglichst feine Verteilung der Arzneimittel angestrebt. Während mit den bekannten Inhalatoren nur eine unzureichende Desagglomeration erzielt wird, werden bei der Benutzung des erfindungsgemäßen Inhalators die Agglomerate weitgehend zerstört.

Der Inhalator gemäß der Erfindung weist eine Kammer für die Aufnahme der Kapsel auf, deren lichte Weite etwa 1,1- bis 2,5-, vorzugsweise 1,1- bis 2,2mal, insbesondere 1,2- bis 1,6mal so groß ist wie der Kapseldurchmesser und deren Länge etwa 1,02- bis 2-, vorzugsweise 1,04- bis 1,8-, insbesondere 1,1- bis 1,6mal so groß ist wie die Länge der Kapsel. Dabei muß die lichte Weite der Kammer kleiner sein als die Länge der Kapsel. Die Lufteintrittsöffnung wird zweckmäßig zentral im Boden der Kammer angeordnet.

Damit die Kapselkappe sie verschließen kann, ist die Öffnung kleiner als der Kapseldurchmesser; ihr Durchmesser beträgt bevorzugt 0,05 bis 0,5 der lichten Weite der Kammer. Sie ist vorzugsweise rund. Die Kammer hat zweckmäßig zylindrische Form, sie kann jedoch auch einen ovalen oder viereckigen Querschnitt besitzen. Der Luftauslaß muß sich in dem der Lufteinlaßöffnung gegenüberliegenden Teil der Kammer befinden und derart gestaltet sein, daß die Kapsel nicht beim Inhalieren an der Auslaßöffnung festgesaugt wird. Um den Verschluß der Auslaßöffnung durch die Kapsel zu verhindern, bestehen verschiedene Möglichkeiten. Beispielsweise kann die der Lufteintrittsöffnung gegenüberliegende Wand der Kapselkammer als Siebplatte ausgestaltet sein oder es werden vorspringende Bauteile vorgesehen, die sich einander soweit nähern, daß ihr Abstand geringer ist als der Kapseldurchmesser. Ferner können eine oder mehrere Öffnungen am oberen Ende der Seitenwände als Auslaßöffnungen vorgesehen sein. Der für das Ausströmen der Luft aus der Kapselkammer zur Verfügung stehende Querschnitt ist zweckmäßig überall größer als die Lufteinlaßöffnung, damit die mit dem Arzneimittel beladene Luft möglichst ungehindert ausströmen kann.

Die in der Kammer mit dem Arzneistoff vermischte Luft wird durch ein Mundstück dem Mund des Anwenders zugeleitet. Das Mundstück, das im allgemeinen röhrenförmig, gegebenenfalls etwas abgeflacht ist, kann axial oder auch in einem Winkel zur Achse der Kammer angeordnet oder seitlich zur Achse der Kammer versetzt sein.

Der erfindungsgemäße Inhalator besteht aus mindestens zwei Teilen. Dabei sind die Teile so gestaltet, daß bei ihrer Trennung die Kammer geöffnet ist und die Kapsel eingelegt beziehungsweise entnommen werden kann. Wenn die Kammer seitlich angeordnet ist, kann eine axial verschiebbare Abdeckung vorgesehen sein, die je nach ihrer Stellung die Kammer öffnet oder schließt und die in geschlossener Stellung einen Teil der Seitenwände der Kammer bildet. Ist der die Kammer enthaltende Teil des Inhalators zylindrisch geformt, kann die Abdeckung der Kammer auch durch eine um die Achse des Inhalators drehbare Hülse bewirkt werden, die in einer Position das Einlegen und Entnehmen der Kapsel gestattet.

Die Kapsel muß für den Inhalationsvorgang nahe ihren beiden Enden geöffnet werden. Dabei dürfen die halbkugelförmigen Kappen der Kapsel nicht beschädigt werden. Das ist deshalb wichtig, weil die Kapsel bzw. Kapselkappe eine Art Ventilfunktion ausübt. Aufgrund der Druckverhältnisse wird die Kapsel gegen die einströmende Luft an die Einlaßöffnung gezogen und verschließt diese. Da der Benutzer weiter am Mundstück saugt, entsteht in der Kapselkammer ein Unterdruck, durch den die Kapsel mit der einströmenden Luft in Richtung auf den Luftauslaß mitgerissen wird. Der nun am Lufteinlaß entstehende Unterdruck bewirkt, daß die Kapsel erneut an die Einlaßöffnung gezogen wird. Der ganze Vorgang wiederholt sich in rascher Folge, solange durch das Mundstück inhaliert wird, und versetzt die Kapsel in starke Vibration.

Eine Ausführungsform der Erfindung ist in den Figuren 1 bis 2 dargestellt.

Fig. 1 zeigt einen Längsschnitt durch den Inhalator.

Fig. 2 ist ein Querschnitt nach Linie A–A in Fig. 1.

Fig. 3 zeigt einen Querschnitt durch einen erfindungsgemäßen Inhalator, bei dem die Kammer seitlich angeordnet und durch eine verschiebbare Platte geöffnet beziehungsweise geschlossen werden kann.

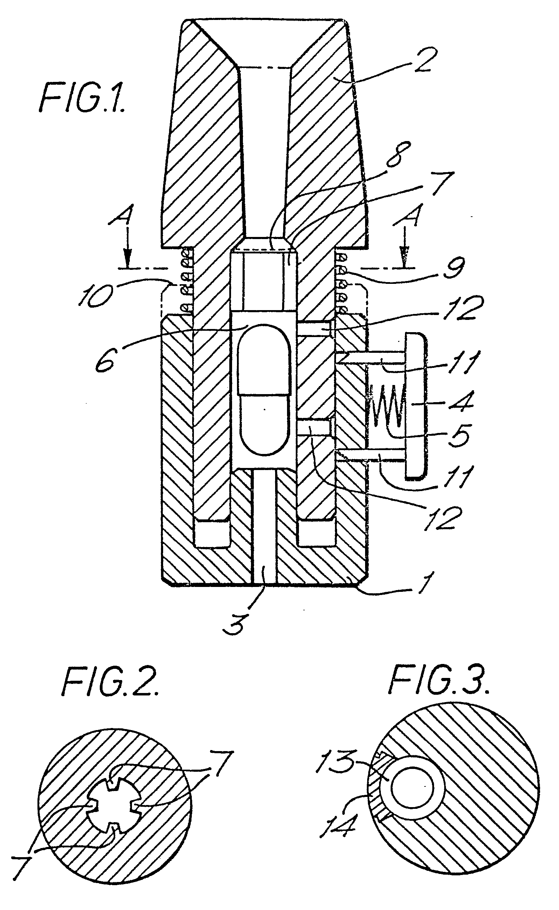

Die in Fig. 1 wiedergegebene Ausführungsform des erfindungsgemäßen Inhalators besteht aus dem Unterteil 1 und dem Mundstück 2, die zusammengesteckt werden. Das Unterteil enthält den Lufteinlaßkanal 3 und eine Schneidvorrichtung 4, die durch ein Federelement 5 in ihrer Normalposition gehalten wird. Das Mundstück 2 enthält die Kapselkammer 6. In die Verlängerung des Kapselraumes ragen Vorsprünge 7, die der Spielraum der Kapsel begrenzen. Eine Siebplatte 8 verhindert, daß z.B. Bruchstücke der Kapsel mit inhaliert werden können. Der Inhalator kann gegen den Druck eines Federelementes 9 axial zusammengedrückt werden, wobei der obere Rand des Unterteils die Position 10 erreicht. In dieser Position können die Messer beziehungsweise Spitzen 11 der Schneidvorrichtung 4 durch die Öffnung 12 in die Kapselkammer 6 eindringen und die dort dann fixierte Kapsel öffnen.

Fig. 2 zeigt die Verlängerung der Kapselkammer mit den Vorsprüngen 7, die den Spielraum der Kapsel auf die eigentliche Kapselkammer 6 begrenzen.

Fig. 3 läßt die seitlich angeordnete Kapselkammer 13 erkennen sowie das axial verschiebbare Element 14, das zum Öffnen und Schließen der Kapselkammer dient und gleichzeitig einen Teil der Seitenwand bildet.

Zur Benutzung des Inhalators nach Fig. 1 werden Unterteil 1 und Mundstück 2 auseinandergezogen, die Kapsel eingelegt und die beiden Inhalatorteile zusammengesteckt. Nach dem Zusammendrücken in Position 10 gegen das Federelement 9 wird die Schneidvorrichtung 4 betätigt und wieder losgelassen. Unter dem Druck des Federelementes 9 geht der Inhalator wieder in die in Fig. 1 dargestellte Position über. Nun wird durch Einatmen durch das Mundstück 2 inhaliert.

**Patentanspruch**

Inhalator für die Inhalation pulverförmiger, insbesondere mikronisierter Arzneimittel aus Kapseln, mit einer länglichen Kapselkammer (6), deren lichte Weite 1,1- bis 2,5mal so groß ist wie der Kapseldurchmesser und deren Länge 1,02- bis 2mal so groß ist wie die Länge der Kapsel, und bei dem mit einem Lufteinlaß (3) im Boden der Kapselkammer (6) und einem Luftauslaß in der Decke der Kapselkammer (6) der Luftstrom axial geführt wird, dadurch gekennzeichnet, daß die Kapselkammer (6) von einem Unterteil (1), das mit einer Schneidvorrichtung (4) versehen ist, die zwei Spitzen bzw. Schneiden (11) aufweist, und einem Mundstück (2) begrenzt wird, wobei Unterteil (1) und Mundstück (2) so ausgebildet sind, daß sie aus der Normalposition axial gegeneinander in die Lochungsposition verschoben werden können, in der die Kapsel fixiert ist und durch Betätigung der Schneidvorrichtung (4) seitlich nahe den beiden Kapselenden gelocht bzw. aufgeschnitten wird.

**Claim**

Inhaler for the inhalation of powdered, more particularly micronised, pharmaceutical compositions from capsules, having an elongate capsule member (6), the internal width of which is 1.1 to 2.5 times the capsule diameter and the length of which is 1.02 to 2 times the length of the capsule, and wherein the current of air is guided axially with an air inlet (3) in the base of the capsule chamber (6) and an air outlet in the top of the capsule chamber (6), characterized in that the capsule chamber (6) is defined by a lower part (1) provided with a cutting device (4) which has two points or cutting edges (11), and a mouthpiece (2), the lower part (1) and mouthpiece (2) being constructed so that they can be displaced axially relative to each other from the normal position into the perforating position in which the capsule is fixed and, by actuation of the cutting device (4), is perforated or cut open laterally close to the two ends of the capsule.

**Revendication**

Inhalateur pour l'inhalation de médicaments pulvérulents, notamment micronisés, renfermés par des capsules, présentant un compartiment longiligne (6) à capsules dont la largeur intérieure représente de 1,1 à 2,5 fois le diamètre de la capsule, et dont la longueur représente de 1,02 à 2 fois la longueur de cette capsule, inhalateur dans lequel le courant d'air est guidé axialement grâce à une admission d'air (3) dans le fond du compartiment (6) à capsules, et à une sortie d'air dans le sommet et de ce compartiment (6) à capsules, caractérisé par le fait que ledit compartiment (6) à capsules est délimité par un embout (2) et par une partie inférieure (1) qui est munie d'un dispositif de sectionnement (4) comportant respectivement deux pointes ou tranchants (11), la partie inférieure (1) et l'embout (2) étant réalisés de telle sorte qu'un coulissement relatif axial puisse leur être imprimé, à partir de la position normale, jusqu'à la position de perforation dans laquelle la capsule est consignée à demeure et est respectivement perforée ou éventrée dans le sens latéral, à proximité des deux extrémités de cette capsule, par un actionnement du dispositif de sectionnement (4).

FIG.1.

FIG.2.

FIG.3.